# EUROPEAN PATENT APPLICATION

(11) **EP 3 363 493 A1**
(43) Date of publication of application: **22.08.2018**
(21) Application number: 18156636.5
(22) Date of filing: 14.02.2018
(51) Int. Cl.: A61N 1/05, A61B 18/14

(54) **INTERLEAVED ABLATION ELECTRODES**

(30) Priority: 15.02.2017 US 201715433509
(71) Applicant: Biosense Webster (Israel) Ltd., Yokneam 2066717 (IL)
(72) Inventor: GOVARI, Assaf, 2066717 Yokneam (IL); ALGAWI, Yehuda, 2066717 Yokneam (IL); ALTMANN, Andres Claudio, 2066717 Yokneam (IL)
(74) Representative: Carpmaels & Ransford LLP

(57) **Abstract**

A medical instrument includes an inflatable balloon and first and second electrodes. The inflatable balloon is coupled to a distal end of a catheter. The first and second electrodes have respective first and second shapes that are interleaved with one another.

## Description

### FIELD OF THE INVENTION

The present invention relates generally to medical ablation techniques, and particularly to design and use of ablation electrodes.

### BACKGROUND OF THE INVENTION

Various types of electrodes may be used in medical procedures, such as in cardiac mapping and/or treatment applications.

For example, U.S. Patent 6,164,283, whose disclosure is incorporated herein by reference, describes a method of treatment of a patient diagnosed with atrial arrhythmia by forming a circumferential conduction block in a region of tissue at a location where a pulmonary vein extends from an atrium. The method includes either forming one such circumferential conduction block around one of the pulmonary vein ostia, forming multiple such circumferential conduction blocks around each one of the pulmonary vein ostia or in subset combinations thereof.

U.S. Patent Application Publication 2013/0274562, whose disclosure is incorporated herein by reference, describes an apparatus for medical diagnosis and/or treatment. The apparatus includes a flexible substrate, an intermediate bus disposed on the flexible substrate, and a plurality of sensing elements disposed on the flexible substrate and coupled to the intermediate bus. The plurality of sensing elements and intermediate bus are disposed on the flexible substrate such that the sensing elements are disposed at areas of minimal strain of the flexible substrate.

U.S. Patent 8,805,466, whose disclosure is incorporated herein by reference, describes a tissue electrode assembly that includes a membrane configured to form an expandable, conformable body that is deployable in a patient. The assembly further includes a flexible circuit positioned on a surface of the membrane and comprising at least one base substrate layer, at least one insulating layer and at least one planar conducting layer. An electrically-conductive electrode covers at least a portion of the flexible circuit and a portion of the surface of the membrane not covered by the flexible circuit.

### SUMMARY OF THE INVENTION

An embodiment of the present invention that is described herein provides a medical instrument including an inflatable balloon and first and second electrodes. The inflatable balloon is coupled to a distal end of a catheter. The first and second electrodes have respective first and second shapes that are interleaved with one another.

In some embodiments, the first and second electrodes are electrically-insulated from one another. In other embodiments, the first and second electrodes are disposed on an outer surface of the inflatable balloon. In yet other embodiments, the first electrode has at least one concave region, and the second electrode has at least one convex region that protrudes into the concave region of the first electrode.

In an embodiment, the first electrode has at least one two-dimensional (2D) depression, and the second electrode has at least one 2D protrusion that protrudes into the 2D depression of the first electrode. In another embodiment, the first electrode is concave and is inscribed by a convex shape, and the second electrode has at least one region that extends into the convex shape that inscribes the first electrode. In yet another embodiment, the first and second electrodes are electrically connected to the distal-end and are configured to receive a signal via the catheter.

In some embodiments, the first and second electrodes are configured to ablate tissue so as to form a contiguous lesion along the tissue.

There is additionally provided, in accordance with an embodiment of the present invention, a method for producing a medical instrument. The method includes coupling an inflatable balloon to a distal end of a catheter. First and second electrodes having respective first and second shapes that are interleaved with one another are disposed on the balloon.

There is additionally provided, in accordance with an embodiment of the present invention, a method for ablation at a target location in tissue of a patient. The method includes inserting into the patient body an inflatable balloon coupled to a distal end of a catheter. The inflatable balloon has disposed thereon first and second electrodes having respective first and second shapes that are interleaved with one another. The distal end of the catheter is navigated to the target location, and the balloon is inflated to make physical contact between the first and second electrodes and the tissue. Electrical ablation signals are applied to the first and second electrodes.

The present invention will be more fully understood from the following detailed description of the embodiments thereof, taken together with the drawings in which:

### BRIEF DESCRIPTION OF THE DRAWINGS

FIG. 1 is a schematic, pictorial illustration of a catheter-based tracking and ablation system, in accordance with an embodiment of the present invention; and
Figs. 2-3 are schematic, side views of balloon assemblies of a catheter, on which electrodes are disposed, in accordance with embodiments of the present invention.

### DETAILED DESCRIPTION OF EMBODIMENTS

### OVERVIEW

Balloon catheters are used in various interventional cardiology procedures, such as in treating arrhythmia, by forming lesions that block electrical conduction along a path of tissue in a patient heart. A lesion that blocks undesired intra-heart electrical signals may be formed using various techniques, such as by applying a radio-frequency (RF) ablation to the tissue at a selected location.

One possible ablation solution is to dispose an array of electrodes on an outer surface of an inflatable balloon that is inserted to the desired ablation site. If, however, such electrodes were to be separated by linearly shaped electrically-insulating regions, discontinuities were likely to remain between the lesions created by the electrodes, thereby allowing undesirable conductance of intra-heart electrical signals. In principle, expensive production techniques may be used for shrinking the width of the electrically-insulating regions between the electrodes. Such techniques, however, may fail to completely block the undesired conductance of the intra-heart electrical signals between the lesions.

Embodiments of the present invention that are described hereinbelow provide improved ablation electrode configurations and associated methods, for forming contiguous lesions that block undesired conductance of the intra-heart electrical signals.

In some embodiments, a balloon catheter comprises an inflatable balloon coupled to a distal end of a catheter. Two or more electrodes are disposed on an outer surface of the balloon. The electrodes are shaped and positioned on the balloon so as to be interleaved with one another. The interleaved configuration helps to avoid gaps between the individual lesions created by the respective electrodes, resulting in reliable contiguous ablation. Several examples of interleaved electrode configurations are described herein. The electrodes are typically separated from one another by an electrically-insulating region having a non-linear boundary.

The disclosed techniques can be used with various production techniques of electrodes on the balloon, such as bonding electrodes on a lump. The interleaved structure of the electrodes allows forming a contiguous lesion without the need to shrink the width of the electrically-insulating region between the electrodes. Therefore, the disclosed techniques enable producing such balloon catheters at affordable cost without compromising performance, and improving the quality of the arrhythmia treatment.

### SYSTEM DESCRIPTION

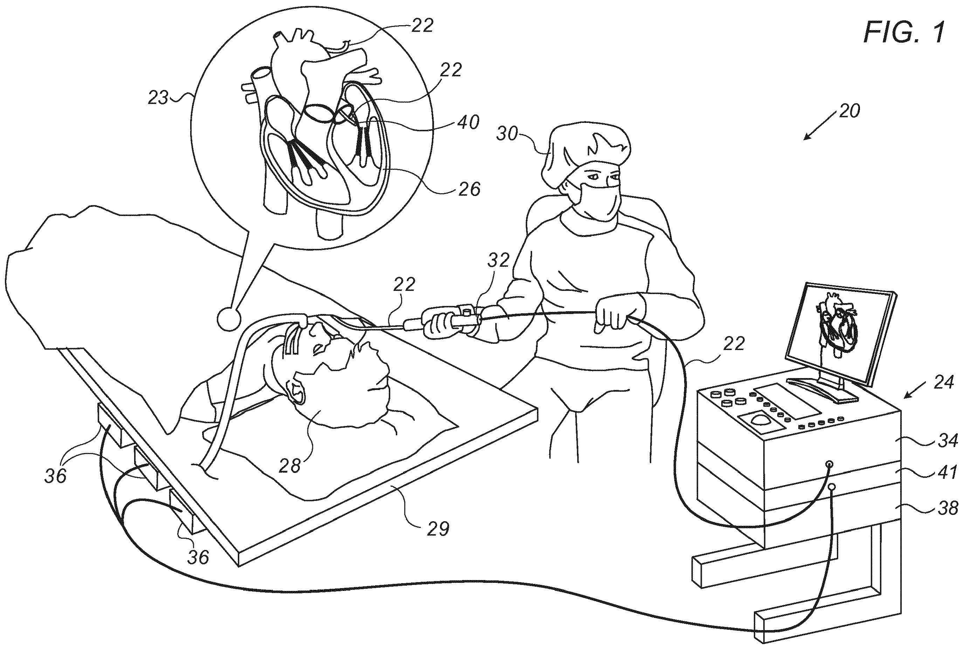

Fig. 1 is a schematic, pictorial illustration of a catheter-based tracking and ablation system 20, in accordance with an embodiment of the present invention. System 20 comprises a catheter 22, in the present example a cardiac catheter, and a control console 24. In the embodiment described herein, catheter 22 may be used for any suitable therapeutic and/or diagnostic purposes, such as ablation of tissue in a heart 26.

Console 24 comprises a processor 41, typically a general-purpose computer, with suitable front end and interface circuits 38 for receiving signals via catheter 22 and for controlling the other components of system 20 described herein.

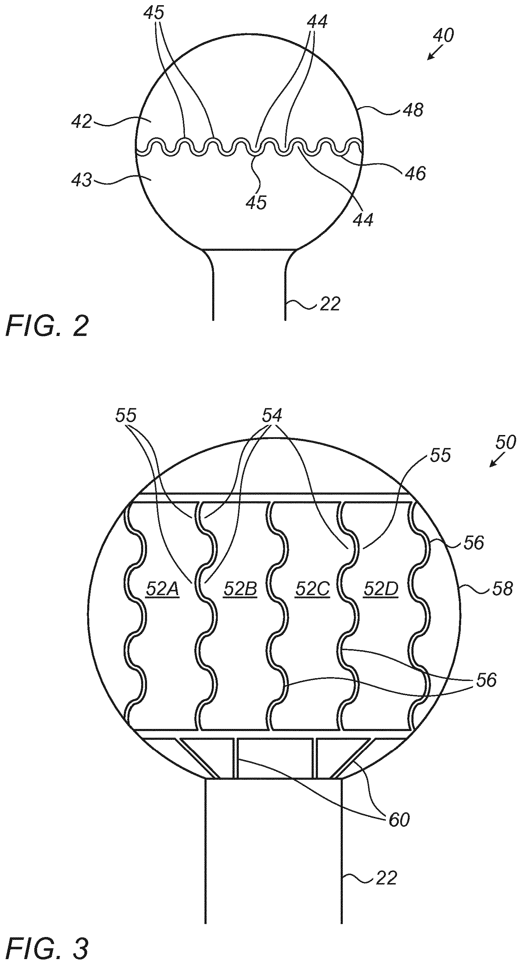

A physician 30 inserts catheter 22 through the vascular system of a patient 28 lying on a table 29. Catheter 22 comprises a balloon assembly 40 fitted at its distal end. Balloon assembly 40 is configured to ablate tissue at a target location of heart 26. Several configurations of ablation balloon assemblies are depicted in detail in Figs. 2 and 3 below. Physician 30 navigates balloon assembly 40 in the vicinity of the target location in heart 26 by manipulating catheter 22 with a manipulator 32 near the proximal end of the catheter as shown in an inset 23. The proximal end of catheter 22 is connected to interface circuitry in processor 41.

In some embodiments, the position of balloon assembly 40 in the heart cavity is measured by a position sensor (not shown) of a magnetic position tracking system. In this case, console 24 comprises a driver circuit 34, which drives magnetic field generators 36 placed at known positions external to patient 28 lying on table 29, e.g., below the patient's torso. The position sensor is configured to generate position signals in response to sensed external magnetic fields from field generators 36. The position signals are indicative of the position of balloon assembly 40 in the coordinate system of the position tracking system.

This method of position sensing is implemented in various medical applications, for example, in the CARTO™ system, produced by Biosense Webster Inc. (Diamond Bar, Calif.) and is described in detail in U.S. Patents 5,391,199, 6,690,963, 6,484,118, 6,239,724, 6,618,612 and 6,332,089, in PCT Patent Publication WO 96/05768, and in U.S. Patent Application Publications 2002/0065455 A1, 2003/0120150 A1 and 2004/0068178 A1, whose disclosures are all incorporated herein by reference.

Processor 41, typically comprises a general-purpose computer, which is programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

### CONTIGUOUS ABLATION USING INTERLEAVED ELECTRODES

Fig. 2 is a schematic side view of balloon assembly 40, in accordance with an embodiment of the present invention. In some embodiments, assembly 40 comprises an inflatable balloon 48 made from polyethylene terephthalate (PET) or any other suitable material.

During the insertion of catheter 22, balloon 48 is contained in a sheath (not shown) in a collapsed position. After being navigated to a target position (e.g., an ostium of a pulmonary vein), balloon 48 is inflated to an expanded position, typically using a bio-compatible saline liquid supplied by system 20, via catheter 22.

In some embodiments, assembly 40 comprises ablation electrodes 42 and 43 disposed on the external surface of the distal and proximal hemispheres of balloon 48, respectively. In an embodiment, after navigating assembly 40 to the target location at heart 26, physician 30 may inflate balloon 48 so as to make physical contact between electrodes 42 and 43 and tissue at the target location. Electrodes 42 and 43 are configured to receive electrical ablation signals, such as radio frequency (RF), via suitable wires that run through catheter 22, and to ablate tissue at the target location in heart 26.

The figure shows balloon 48 in its expanded position, ready to receive ablation signals for via catheter 22 for ablating the tissue at the target location, using electrodes 42 and 43. After ablating the tissue, balloon 48 is deflated and re-inserted to the sheath so that physician 30 may safely retract assembly 40 out of the body of patient 28.

As can be seen in the figure, the shapes of electrodes 42 and 43 are non-linear. Each electrode has a reentrant shape comprising a repeating structure of concave and convex shapes. The electrodes are interleaved with one another by arranging a convex region of one electrode to inside a concave region of the other electrode and *vice versa.* For example, a convex region 44 of electrode 42 faces a concave region 45 of electrode 43 so that convex region 44 protrudes into concave region 45.

Put in another way, electrode 42 may have at least one two-dimensional depression, and electrode 43 may have at least one two-dimensional protrusion that protrudes into the depression of electrode 42.

In some embodiments, electrodes 42 and 43 are separated from one another by an electrically-insulating region 46 having a non-linear boundary formed by the reentrant shapes of electrodes 42 and 43. In this embodiment, region 46 is contiguous and physically separates between electrodes 42 and 43 across the perimeter of balloon 48, so that electrodes 42 and 43 are electrically insulated from one another. In this embodiment, electrodes 42 and 43 receive the ablation electrical signals via catheter 22 in parallel.

The reentrant shape and interleaved arrangement of electrodes 42 and 43 allows formation of a contiguous lesion pattern that blocks propagation of undesired electrical impulses across the target region of heart 26. In other words, the non-linear shape of boundary region 46 reduces the likelihood of a discontinuity remaining between the lesions formed by electrodes 42 and 43. In yet other words, a first electrode (e.g., electrode 42) is concave and is inscribed by a convex shape, whereas a second electrode (e.g., electrode 43) has at least one region that extends into the convex shape that inscribes the first electrode.

In alternative embodiments, electrodes 42 and 43 are electrically interconnected at or near the distal end of the catheter. In this embodiment, both electrodes may be driven with a single ablation signal. In an embodiment, electrode 42 receives the electrical signal from catheter 22, via electrode 43.

In the example of Fig. 2, the shape of regions 44 and 45 is rounded. In alternative embodiments, regions 44 and 45 may have any other suitable shape, such as V-Shaped or double V-shape.

### PRODUCING AN INFLATABLE BALLOON CATHETER HAVING INTERLEAVED ELECTRODES

As explained above, in some embodiments balloon assembly 40 may be produced by disposing electrodes 42 and 43 on balloon 48 such that their respective shapes are interleaved with one another, and then coupling assembly 40 to the distal end of catheter 22 so that assembly 40 will have the functionalities described above (e.g., inflating/deflating and ablating). In other embodiments, disposing electrodes 42 and 43 on balloon 48 may be carried out after coupling balloon 48 to the distal end of catheter 22.

Fig. 3 is a schematic, side view of a balloon assembly 50, in accordance with an alternative embodiment of the present invention. Assembly 50 may replace, for example, assembly 40 of Fig. 2 above. In some embodiments, assembly 50 comprises an inflatable balloon 58 that may be substantially similar to balloon 48 depicted in Fig. 2 above.

In some embodiments, ablation electrodes 52A, 52B, 52C, and 52D are disposed next to one another on the external surface of balloon 58. Each of electrodes 52A-52D has a reentrant shape comprising convex regions 54 and concave regions 55 that interleave with the corresponding regions of an adjacent electrode. For example, in an embodiment convex regions 54 of electrode 52B extends into concave regions 55 of electrode 52A.

In some embodiments, neighboring electrodes among electrodes 52A-52D (e.g., electrodes 52C and 52D) are separated by respective electrically-insulating regions 56 having non-linear boundaries formed by the reentrant shape of the electrodes.

In an embodiment, electrodes 52A-52D receive the ablation electrical signals via catheter 22 and electrical conductors 60. Electrodes 52A-52D may be electrically connected to catheter 22 in series or alternatively in parallel.

The example configuration shown in Figs. 2 and 3 are chosen purely for the sake of conceptual clarity. In alternative embodiments, the disclosed techniques may use other suitable shapes of electrodes that are interleaved in one another and thus enable formation of a contiguous lesion in the target location. Moreover, the disclosed techniques are not limited to balloon assemblies, and can be used with other suitable distal-end assemblies that comprise ablation electrodes.

It will be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

### ASPECT OF THE INVENTION

1. A method for ablation at a target location in tissue of a patient, the method comprising:
   inserting into the patient body an inflatable balloon coupled to a distal end of a catheter, wherein the inflatable balloon has disposed thereon first and second electrodes having respective first and second shapes that are interleaved with one another;
   navigating the distal end of the catheter to the target location, and inflating the balloon to make physical contact between the first and second electrodes and the tissue; and
   applying electrical ablation signals to the first and second electrodes.

## Claims

1. A medical instrument, comprising:
an inflatable balloon coupled to a distal end of a catheter; and
first and second electrodes, having respective first and second shapes that are interleaved with one another.

2. The medical instrument according to claim 1, wherein the first and second electrodes are electrically-insulated from one another.

3. The medical instrument according to claim 1, wherein the first and second electrodes are disposed on an outer surface of the inflatable balloon.

4. The medical instrument according to claim 1, wherein the first electrode has at least one concave region, and wherein the second electrode has at least one convex region that protrudes into the concave region of the first electrode.

5. The medical instrument according to claim 1, wherein the first electrode has at least one two-dimensional (2D) depression, and wherein the second electrode has at least one two-dimensional protrusion that protrudes into the 2D depression of the first electrode.

6. The medical instrument according to claim 1, wherein the first electrode is concave and is inscribed by a convex shape, and wherein the second electrode has at least one region that extends into the convex shape that inscribes the first electrode.

7. The medical instrument according to claim 1, wherein the first and second electrodes are electrically connected to the distal-end and are configured to receive a signal via the catheter.

8. The balloon assembly according to claim 1, wherein the first and second electrodes are configured to ablate tissue so as to form a contiguous lesion along the tissue.

9. A method for producing a medical instrument, the method comprising:
coupling an inflatable balloon to a distal end of a catheter; and
disposing on the balloon first and second electrodes, having respective first and second shapes that are interleaved with one another.

10. The method according to claim 9, wherein the first and second electrodes are electrically-insulated from one another.

11. The method according to claim 9, wherein disposing the first and second electrodes comprises separating the first and second electrodes from one another by an electrically-insulating region having a non-linear boundary.

12. The method according to claim 9, wherein the first electrode has at least one concave region, and wherein the second electrode has at least one convex region that protrudes into the concave region of the first electrode.

13. The method according to claim 9, wherein the first electrode has at least one two-dimensional depression, and wherein the second electrode has at least one two-dimensional protrusion that protrudes into the depression of the first electrode.

14. The method according to claim 9, wherein the first electrode is concave and is inscribed by a convex shape, and wherein the second electrode has at least one region that extends into the convex shape that inscribes the first electrode.

15. The method according to claim 9, wherein disposing the first and second electrodes comprises electrically connecting the first and second electrodes to the distal end.
